Europäisches Patentamt

European Patent Office

Office européen des brevets

(19)

(11) Veröffentlichungsnummer : **0 163 223**
**B1**

# (12) EUROPÄISCHE PATENTSCHRIFT

(45) Veröffentlichungstag der Patentschrift :
04.03.87

(21) Anmeldenummer : 85106134.1

(22) Anmeldetag : 18.05.85

(51) Int. Cl.⁴ : **C 07 C121/453**

(54) **Verfahren zur Herstellung von Carnitinnitrilchlorid.**

(30) Priorität : 26.05.84 DE 3419723

(43) Veröffentlichungstag der Anmeldung :
04.12.85 Patentblatt 85/49

(45) Bekanntmachung des Hinweises auf die Patenterteilung : 04.03.87 Patentblatt 87/10

(84) Benannte Vertragsstaaten :
BE CH DE FR GB IT LI NL

(56) Entgegenhaltungen :
EP-A- 0 060 595
US-A- 3 151 149
CHEMICAL ABSTRACTS, Band 57, Nr. 11, 26. November 1962, Columbus, Ohio, USA; Spalte 13621 a-b
CHEMICAL ABSTRACTS, Band 98, Nr. 7, 14. Februar
1983, Columbus, Ohio, USA; Abstract Nr. 54493e;
CHEMICAL ABSTRACTS, Band 55, Nr. 9, 1. Mai 1961,
Columbus, Ohio, USA; E. STRACK et al. "Preparation
of L-carnitine and its isomers", Spalte 8302 c-e

(73) Patentinhaber : BASF Aktiengesellschaft
Carl-Bosch-Strasse 38
D-6700 Ludwigshafen (DE)

(72) Erfinder : Puetter, Herrmann, Dr.
Haardter Strasse 1
D-6730 Neustadt (DE)
Erfinder : Roske, Eckhard
Hilliensheimer Strasse 4
D-6700 Lufwigshafen (DE)
Erfinder : Pander, Hans Joachim
Hochdorfer Strasse 19
D-6701 Roedersheim-Gronau (DE)

## Beschreibung

Diese Erfindung betrifft ein Verfahren zur Herstellung von Carnitinnitrilchlorid durch Umsetzung von 3-Chlor-2-hydroxibutyronitril mit Trimethylamin in einem Lösungsmittel bei höherer Temperatur und unter Druck.

Carnitinnitrilchlorid ist ein wichtiges Zwischenprodukt für die Herstellung von Carnitin und Derivaten des Carnitins, die als Pharmazeutika verwendet werden.

Aus der US-PS-3 135 788 ist bekannt, daß man Carnitinnitrilchlorid durch Umsetzung von Epichlorhydrin mit Trimethylaminhydrochlorid und Natriumcyanid herstellen kann. Bei dieser Reaktion, die man in wäßrigem Medium bei Temperaturen bis 50 °C oder in Methanol oder Ethanol durchführt, läßt sich die Bildung von Nebenprodukten durch Mehrfachquaternisierung nicht ausschließen. Störend ist auch der Zwangsanfall an Natriumchlorid. Außerdem wird das als Zwischenprodukt gebildete 3-Chlor-2-hydroxipropyltrimethylammoniumchlorid in alkalischem Medium leicht hydrolysiert.

Man hat das Carnitinnitril auch schon durch Umsetzung von 3-Chlor-2-hydroxibutyronitril mit Trimethylamin in wäßriger Lösung hergestellt (EP-A-0 060 595). Bei dieser Arbeitsweise ist die Bildung von Trimethylaminhydrochlorid als Nebenprodukt unvermeidbar, wodurch sich die Ausbeute vermindert.

Es wurde nun gefunden, daß man bei der Herstellung von Carnitinnitrilchlorid durch Umsetzung von 3-Chlor-2-hydroxibutyronitril mit Trimethylamin erheblich vorteilhaftere Ergebnisse erzielt, wenn man die Umsetzung in einem organischen Lösungsmittel, das keine Hydroxylgruppen enthält, bei Temperaturen von 60 bis 140 °C und unter Druck vornimmt.

Nach dem neuen Verfahren erhält man überraschenderweise keine Nebenprodukte, wie sie z. B. bei der bekannten Umsetzung in Wasser entstehen.

Als organische Lösungsmittel, die keine Hydroxylgruppen enthalten, kommen z. B. aprotische Lösungsmittel, wie Toluol, Dioxan, Cyclohexan, Monochlorbenzol, Tetrahydrofuran, Dimethylformamid, Aceton oder Dekalin in Betracht.

Man benötigt auf 100 Teile 3-Chlor-2-hydroxibutyronitril z. B. 200 bis 500 Teile des Lösungsmittels. Man kann als Lösungsmittel auch das Trimethylamin selbst verwenden, indem man einen Überschuß an Trimethylamin, wie die 5- bis 10fache molare Menge, bezogen auf das 3-Chlor-2-hydroxybutyronitril, einsetzt. Nimmt man anstelle von Trimethylamin eines der obengenannten Lösungsmittel, so wird das Trimethylamin z. B. in der 1,2- bis 2fachen molaren Menge, bezogen auf 1 Mol 3-Chlor-2-hydroxybutyronitril, angewendet.

Das erfindungsgemäße Verfahren wird bei Temperaturen von 60 bis 140 °C, vorzugsweise bei 80 bis 120 °C und unter einem Druck von 5 bis 100 bar, vorzugsweise 10 bis 40 bar durchgeführt. Man geht dabei zweckmäßigerweise so vor, daß man die Umsetzung in einem Rührautoklaven bei dem durch Erhitzen auf die Umsetzungstemperatur entstehenden Eigendruck vornimmt. Die Reaktion ist nach etwa 3 bis 30 Stunden beendet. Man arbeitet das Reaktionsgemisch z. B. auf, indem man das ausgefallene Kristallisat absaugt und trocknet.

Man erhält Carnitinnitrilchlorid in Ausbeuten von etwa 95 % und höher und mit hoher Selektivität. Wegen seiner hohen Reinheit ist es hervorragend für die Herstellung von Carnitinamid und Carnitin geeignet.

Beispiel 1

36,2 g (0,3 Mol) 3-Chlor-2-hydroxibutyronitril wurden in einem 300 ml-Rührautoklav mit 118 g (2,0 Mol) wasserfreiem Trimethylamin unter Rühren bei 100 °C und ca. 20 bar Eigendruck umgesetzt. Nach 24 h wurde abgekühlt, der Trimethylaminüberschuß abgegast und das Kristallisat ausgetragen und getrocknet. Man erhielt 50,5 g eines trimethylaminhydrochloridfreien, NMR-reinen Carnitinnitrilchlorids (3-Nitrilo-2-hydroxipropyl-trimethylammoniumchlorid) in 94,3 %iger Ausbeute.

Beispiel 2

36,2 g (0,3 Mol) 3-Chlor-2-hydroxibutyronitril wurden in 100 ml Toluol gelöst. Die Lösung wurde in einem 300 ml Rührautoklav mit 35,4 g (0,6 Mol) wasserfreiem Trimethylamin unter Rühren bei 100 °C und ca. 15 bar Eigendruck umgesetzt. Nach 24stündiger Versuchsdauer wurde der abgekühlte Kristallbrei ausgetragen, abgesaugt und getrocknet. Es wurden 52,2 g NMR-reines $Me_3N \cdot HCl$-freies Carnitinnitrilchlorid erhalten. Die Ausbeute betrug 97,5 %, bezogen auf 3-Chlor-2-hydroxibutyronitril.

Beispiel 3

36,2 g (0,3 Mol) 3-Chlor-2-hydroxibutyronitril wurden in 100 ml Dioxan gelöst. Die Lösung wurde in einem 300 ml Rührautoklav mit 29,5 g (0,5 Mol) wasserfreiem Trimethylamin unter Rühren bei 100 °C und ca. 40 bar Eigendruck umgesetzt. Nach 24stündiger Versuchsdauer und Abkühlung wurde der Kristallbrei ausgetragen, abgesaugt und getrocknet: Man erhielt 51,4 g $Me_3N \cdot HCl$-freies, NMR-reines Carnitinnitrilchlorid, entsprechend einer Ausbeute von 96 % bezogen auf 0,3 Mol 3-Chlor-2-hydroxibutyronitril.

Vergleichsbeispiel

239 g (2,0 Mol) 3-Chlor-2-hydroxibutyronitril wurden mit 442 g 40 %iger wäßriger Trimethylaminlösung bei 40 °C innerhalb von ~ 5 h unter Rühren und schwachem Rückfluß umgesetzt. Der Rückflußkühler wurde mit einem Kühlmedium mit der Temperatur von — 20 °C betrieben. Die wäßrige Reaktionslösung wurde im Rotationsverdampfer eingedampft, das Kristallisat abgetrennt und getrocknet. Es enthielt 12 % Me₃N · HCl. Die Ausbeute betrug 83 %, bezogen auf 3-Chlor-2-hydroxibutyronitril.

**Patentansprüche**

1. Verfahren zur Herstellung von Carnitinnitrilchlorid durch Umsetzung von 3-Chlor-2-hydroxibutyronitril mit Trimethylamin, dadurch gekennzeichnet, daß man die Umsetzung in einem organischen Lösungsmittel, das keine Hydroxylgruppen enthält, bei Temperaturen von 60 bis 140 °C und unter Druck vornimmt.

2. Verfahren nach Anspruch 1, dadurch gekennzeichnet, daß man als Lösungsmittel ein aprotisches Lösungsmittel verwendet.

3. Verfahren nach Anspruch 1, dadurch gekennzeichnet, daß man als Lösungsmittel Toluol, Dioxan oder Cyclohexan verwendet.

4. Verfahren nach Anspruch 1, dadurch gekennzeichnet, daß man als Lösungsmittel Trimethylamin verwendet.

**Claims**

1. A process for the preparation of carnitine nitrilochloride by reacting 3-chloro-2-hydroxybutyronitrile with trimethylamine, wherein the reaction is carried out in an organic solvent which does not contain any hydroxyl groups, at a temperature of from 60 to 140 °C and under superatmospheric pressure.

2. A process as claimed in claim 1, wherein an aprotic solvent is used as the solvent.

3. A process as claimed in claim 1, wherein toluene, dioxane or cyclohexane is used as the solvent.

4. A process as claimed in claim 1, wherein trimethylamine is used as the solvent.

**Revendications**

1. Procédé de préparation du chlorure de carnitine-nitrile par réaction du chloro-3 hydroxy-2 butyro-nitrile avec la triméthyl-amine, caractérisé en ce que la réaction est réalisée entre 60 et 140 °C sous pression accrue dans un solvant organique exempt de groupes oxhydryle.

2. Procédé suivant la revendication 1, caractérisé en ce que le solvant est un solvant aprotique.

3. Procédé suivant la revendication 1, caractérisé en ce que le solvant est choisi parmi le toluène, le dioxanne et le cyclohexane.

4. Procédé suivant la revendication 1, caractérisé en ce que le solvant est la triméthyl-amine.